(19) Europäisches Patentamt
European Patent Office
Office européen des brevets

(11) Publication number: **0 015 537**
**B1**

(12) **EUROPEAN PATENT SPECIFICATION**

(45) Date of publication of patent specification: **01.12.82**

(51) Int. Cl.³: **C 07 C 51/347, C 07 C 57/02, B 01 J 31/24, B 01 J 31/28**

(21) Application number: **80101086.9**

(22) Date of filing: **04.03.80**

(54) **Process for the preparation of dienoic acids.**

(30) Priority: **08.03.79 IT 2082479**

(43) Date of publication of application:
**17.09.80 Bulletin 80/19**

(45) Publication of the grant of the patent:
**01.12.82 Bulletin 82/48**

(84) Designated Contracting States:
**BE DE FR GB NL**

(56) References cited:
**FR - A - 1 582 621**
**SU - A - 177 879**
**US - A - 3 723 500**

(73) Proprietor: **Montedison S.p.A.**
**Patents & Licensing Dept. Foro Buonaparte, 31**
**P.O. Box 10528**
**I-20121 Milan (IT)**

(72) Inventor: **Chiusoli, Gian Paolo**
**18bis, Via Solferino**
**Parma (IT)**
Inventor: **Giroldini, William**
**4, Via Col di Lana**
**Bibbiano (reggio Emilia) (IT)**
Inventor: **Salerno, Giuseppe**
**27, Borgo San Giuseppe**
**Parma (IT)**

(74) Representative: **Schmied-Kowarzik, Volker, Dr. et al,**
**Patentanwälte Dr. V. Schmied-Kowarzik Dipl.-Ing. G. Dannenberg Dr. P. Weinhold Dr. D. Gudel Dipl.-Ing. S. Schubert Siegfriedstrasse 8**
**D-8000 München 40 (DE)**

**EP 0 015 537 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of dienoic acids

The present invention relates to the preparation of dienoic acids having the two double bonds placed in position "3,5" with respect to the carboxylic carbon atom starting from salts of 3-butenoic acid and vinyl halides in the presence of complexed catalysts of rhodium or nickel.

The products thus obtained in themselves find interesting industrial applications as intermediates for organic synthesis, with a special consideration for the so-called "fine chemical" products like for instance sorbic acid (for alimentary preserves, as additives for rubbers). Moreover, by exploiting the reactivity of the double bonds present in the molecule, it is possible to prepare polymers. For instance, it is possible to obtain by conventional simple hydrogenation of the double bonds, known acids with applicational fields such as plasticizers, solvents or additives, as capronic acid by hydrogenation of 3,5-hexadienoic acid for the rubber industries or for varnishes.

The Applicant does not know of any description or suggestion in the prior art of the reaction that is exploited for the process of this invention.

According to the known prior art, however by means of reactions not relevant to the reaction of this invention, there may in fact be obtained 2,6-dienoic acids using alternative processes based on the Wittig synthesis starting from $\gamma$-$\delta$-unsaturated aldehydes which reacted with carboalkoxy - triphenyl - phosphoranes.

However, techniques of this kind require the preliminary preparation of the $\gamma,\delta$-unsaturated aldehydes which are difficult to prepare and are not readily available as well as the stoichiometric use of phosphorated intermediates that are objectionable from the point of view of the compatibility of the effluents with the present-day environmental protection requirements, etc. with the corresponding economical burdens that make these techniques substantially unpractical from the point of view of the industry.

Finally, there are known catalytic processes for the preparation of dienoic acids by reaction of unsaturated halides (vinyl-, allyl halides) with acetylene and carbon oxide in hydroxylated solvents, in the presence of nickel-carbonyl or precursors of same.

They are all methods having a low catalytic activity and moreover have industrially a lesser interest due to the use of nickel-carbonyl derivatives which are not suited for industrial applications because of their high toxicity.

Moreover, the unsaturated acids obtained according to that technique, since they have the double bonds in a prevailingly not-conjugated position, must subseqently be isomerized if dienoic acids with conjugated double bonds are desired.

Thus, the object of this invention is that of providing a simple and cheap process for the preparation of dienoic acids, having in particular the double bonds in a conjugated position, that is free of the previously indicated drawbacks of the prior art.

This object is achieved, according to this invention, by a process as defined in Claim 1.

The reaction may for instance be represented by the following equation for the 3,5-hexadienoic acid:

$$1)\ CH_2{=}CHX + CH_2{=}CH{-}CH_2{-}COOM \xrightarrow{cat.} CH_2{=}CH{-}CH{=}CH{-}CH_2{-}COOH + MK.$$

This reaction develops analogously also for the other possible dienoic acids.

In the above equation, the symbols are as defined in Claim 1.

The reaction is conducted in an organic solvent capable of dissolving at least partially, either alone or in admixture with minor quantities of water, the salts of 3-butenoic acid.

Solvents suitable for the purpose have proved to be: hydrocarbons, ethers, esters, nitriles, amides, alcohols, ketones, preferably having a boiling point below 180°C.

Just for indicative purposes and for ease of separation of the reaction products, it would be convenient to operate with solvents having a boiling point below about 150°C.

Thus, amongst the etheral solvents may be chosen: butyl ether, and anisole; amongst the esters: ethyl acetate; amongst the nitriles: acetonitrile; amongst the amides: dimethylformamide; amongst the alcohols: ethyl alcohol; amongst the ketones: acetone and methylethylketone; amongst the hydrocarbons: toluene.

The catalyst is chosen from:

A) a monovalent rhodium complex with hydrocarbyl phosphines having the formula:

$$RhCl(PR''_3)_x \cdot (ol)_y$$

wherein R″ represents a hydrocarbyl group having up to 8 carbon atoms; "ol" represents a simple olefine having from 2 to 8 carbon atoms, or chelating olefine having from 6 to 8 carbon atoms; $x$ is an integer comprised between 1 and 3, while y is an integer chosen from between 0 and 2, provided that x+y=3; and

B) a zerovalent nickel complex with the same phosphines as indicated above having formula:

$$Ni(PR'')_z$$

wherein $R''$ has the meaning already given; and $z$ is an integer to be chosen between 3 and 4.

Said Rh- and Ni-complexes are by themselves known and available on the market, or

they may be obtained according to conventional methods, for instance by reduction of nickel or rhodium salts in the presence of the bonder (phosphine).

More particularly, effective results have been achieved by the use of complexes in which *R''* is chosen from butyl, phenyl and anisyl; the olefine is chosen from ethylene and cyclooctene and the chelating olefine is 1,5-hexadiene or 1,5-cyclooctadiene.

As to the starting products, 3-butenoic acid is a compound industrially known as an intermediate, while the vinyl halides, likewise known, may be prepared according to known or conventional techniques.

The vinyl halides that are most interesting for the purposes of the reaction, are those of the trans-type, especially when using the rhodium catalysts for which they are particularly effective.

Effective vinyl halides have proved to be: vinyl bromide, styryl bromide and styryl bromides substituted in the aromatic nucleus.

Amongst the catalysts, RhCl (P $Ph_3)_3$ and Ni $(PPH_3)_3$ proved likewise effective.

The reactants are used in substantially stoichiometric molar ratios, while their concentration in the solvent is not critical for the purposes of the reaction.

It is also possible to operate with an excess of salt of 3-butenoic acid.

As already previously stated, the reaction is a catalytic one; catalyst quantities of the order of at least 0.1 millimols per litre of the reaction mixture are already sufficient and may go up to 100 millimoles per litre.

As stated above, in the vinyl halide of formula (1) R—CH=CHX, *R* represents a hydrogen atom or a hydrocarbyl group having up to 20 carbon atoms. Said hydrocarbyl group may also in its turn be substituted with groups or atoms that be inert under the foreseen reaction conditions without interfering with them.

Compatible substituents would be, for instance, CN, $OCH_3$ and $OCOCH_3$.

The desired product is separated according to conventional techniques by acidification ($H_2SO_4$) after distillation of the solvent, and extraction.

According to an effective form of embodiment, the process of this invention is conducted in the following way.

Into a closed reactor and under a nitrogen atmosphere at atmospheric pressure, there are introduced: the catalytic complex, the salt of 3-butenoic acid and the vinyl halide, and finally the chosen solvent. The solution thus obtained is maintained at the pre-established temperature for the required time. The mixture is then treated with a diluted inorganic acid. The organic layer is then separated by extraction with a solvent (ethyl ether).

By treatment of the organic extract with aqueous $Na_2CO_3$ and by a successive new acidification and extraction of the new aqueous phase, there is separated the acid part from which the acids are in their turn separated by distillation, etc.

The process, thanks to the mild operational conditions, appears as particularly convenient.

Other advantages consist in the availability of the starting compounds and in the selectivity of the reaction.

The invention will now be described in more detail by the following examples which are however given for merely illustrative purposes.

Example 1 containing also the preparation of sorbic acid from 3,5-hexadienoic acid. Ph stands for phenyl.

Example 1:

Into a 250 cc flask were introduced under a nitrogen atmosphere 9 g of potassium 3-butenoate ($7,2.10^{-2}$ mols), 7.8 g of vinyl bromide ($7,2.10^{-2}$ mols) 0.3 g of $RhCl(PPh_3)_3$ ($3,3.10^{-4}$ mols) and lastly 90 cc of ethyl alcohol.

This mixture was then heated to a temperature of 85°C for about 48 hours.

The raw product was thereupon decomposed with diluted sulphuric acid and then extracted with ether.

By a conventional treatment of the reaction mixture with a caustic soda solution and by subsequent acidification, there were obtained 2.5 g of 3,5-hexadienoic acid.

By further treatment of the 3,5-hexadienoic acid with hot caustic soda at about 100°C, according to conventional methods, by isomerization there was obtained the formation of the sodium salt of sorbic acid, that is of 2,4-hexadienoic acid.

Example 2:

Following the same procedure as that described in example 1 into a 100 cc flask there were placed 4.1 g of potassium 3-butenoate ($3,3.10^{-2}$ mols), 6 g of styryl trans bromide (I) ($3,3.10^{-2}$ mols), 0.1 g of $RhCl(PPh_3)_3$ ($1,1.10^{-4}$ mols) and 30 cc of ethyl alcohol.

This mixture was thereupon heated up to 85°C (under nitrogen atmosphere for about 48 hours.

After decomposition with diluted sulphuric acid and after extraction with ether, there were obtained, by the given methods, about 4 g of 6-phenyl-3,5-hexadienoic acid, as a mixture of 3-CIS-5-TRANS and 3-TRANS-5-TRANS isomers.

Example 3:

According to the same procedure described in example 1, into a 100 cc flask were placed to react: 4.1 g of potassium 3-butenoate ($3,3.10^{-2}$ mols), 6 g of styryl bromide, a CIS-TRANS mixture, ($3,3.10^{-2}$ mols), 2.9 g of Ni $(PPh_3)_3$ ($3,3.10^{-2}$ mols) and 40 cc of ethyl alcohol.

This mixture was thereupon heated up to 105°C under nitrogen atmosphere, for about 2 hours. Operating as in example 1, there were obtained 2,2 g of 6-phenyl-3,5-hexadienoic

acid, a mixture of isomers containing double CIS and TRANS bonds.

Example 4

By operating in the same way as that of example 2, styryl chloride (I) trans in substitution of the styryl bromide was used. Thereby was obtained a mixture of 6 - phenyl - 3,5 - hexadienoic acids.

Example 5

It was operated as in example 1, but using 1 - bromo - 1 - octene instead of vinyl bromide.

Thereby was obtained a mixture of stereoisomers of 3,5-dodecadienoic acid.

The above mentioned radicals have especially the following meaning:

R contains 1 to 20, preferably 1 to 12, carbon atoms, as, for example, alkyl radicals, such as

methyl, ethyl, propyl, i - propyl, butyl, i - butyl, pentyl, hexyl, i - hexyl, octyl, i - octyl, decyl, i - decyl, dodecyl, tetradecyl, octadecyl; cycloalkyl with 4 to 8 carbon atoms (if desired substituted by lower alkyl), e.g. cyclopentyl, cyclohexyl, cycloheptyl; aryl with 6 to 12 carbon atoms, e.g. phenyl, tolyl, xylyl, ethylphenyl, naphthyl, methylnaphthyl; aralkyl with 7 to 10 carbon atoms, e.g. benzyl, phenethyl, phenylpropyl; inert substituents are e.g. CN, O—($C_1$—$C_4$ alkyl), OCO—($C_1$—$C_4$ alkyl) (the alkyl radicals may be defined as above);

R′ is an alkyl group with 1 to 12, especially 1 to 6, carbon atoms, e.g. methyl, ethyl, propyl, i-propyl, butyl, i-butyl, pentyl, hexyl, i-hexyl, but also octyl, i-octyl, decyl, i-decyl, dodecyl;

R″ is an alkyl, aryl or aralkyl group having 1 to 8 carbon atoms (see therefor the above enumerated radicals).

"ol" is preferably a $C_2$—$C_8$ aliphatic or $C_4$—$C_8$ cycloaliphatic monoolefine or an aliphatic or cycloaliphatic diolefine having 6 to 8 carbon atoms.

**Claims**

1. A process for the preparation of 3,5-dienoic acids having double bonds in a conjugated position, characterized in that a vinyl halide of the formula: R—CH=CHX, wherein *R* stands for a hydrogen atom or a hydrocarbyl group having up to 20 carbon atoms, optionally substituted by substituents inert under the reaction conditions, and where *X* is a halogen chosen from chlorine and bromine, is reacted with a salt of 3-butenoic acid of the formula: $CH_2$=CH—$CH_2$—COOM, wherein *M* represents a metal chosen from: Na, K, 1/2 Ca, 1/2 Mg or represents a $NR'_4$ group wherein R′ represents alkyl groups having independently up to 12 carbon atoms, in an organic solvent capable of dissolving at least partially, either alone or in admixture with minor quantities of water, the salts of 3-butenoic acid and under an inert atmosphere, at a temperature between 50°C and 100°C in the case of the use of complexed phosphinic catalysts of rhodium, and between 70°C and 120°C in the case of the use of complexed phosphinic catalysts of nickel and at atmospheric pressure, in the presence of a catalyst chosen from the phosphinic complexes of rhodium and nickel, having the formulae:

$$RhCl(PR''_3)_x \cdot (ol)_y \text{ and } Ni(PR''_3)_z$$

where R″ stands for a hydrocarbyl group having up to 8 carbon atoms; "ol" represents a simple olefine having 2 to 8 carbon atoms or a chelating olefine having 6 to 8 carbon atoms, and where *x* is an integer comprised between 1 and 3, *y* is an integer comprised between 0 and 2 provided that x+y=3, and z is an integer comprised between 3 and 4.

2. A process according to claim 1, characterized in that the organic solvent is a compound chosen from: hydrocarbons, ethers, esters, nitriles, amides, alcohols and ketones, preferably having a boiling point below 180°C.

3. A process according to claim 2, characterized in that the organic solvent is chosen from amongst: ethyl alcohol, butyl ether, anisole, ethyl acetate, acetonitrile, dimethylformamide, acetone, methylethylketone, toluene.

4. A process according to claims 2 and 3, characterized in that the organic solvent is used in admixture with minor quantities of water.

5. A process according to any one of the preceding claims, characterized in that the rhodium catalyst or nickel catalyst, as defined in claim 1, is a complex with a phosphine $P(R'')_3$ wherein *R″* is chosen from: butyl, phenyl and anisyl groups and wherein the olefine "ol" is chosen from: ethylene, cyclooctene, 1,5-hexadiene and 1,5-cyclooctadiene.

6. A process according to any one of the preceding claims, characterized in that the vinyl halide is chosen from vinyl bromide and styryl bromide.

7. A process according to any one of the preceding claims, characterized in that the catalyst is chosen from $RhCl(PPh_3)_3$ and $Ni(PPh_3)_3$.

8. A process according to any one of the preceding claims, characterized in that the vinyl halide and the salt of the 3-butenoic acid are reacted in substantially equimolar ratios.

9. A process according to any one of the preceding claims, characterized in that the complexed rhodium or nickel catalyst is used in quantities at least equal to 0.1 millimols per litre of reacting mass, up to 100 millimols per litre of reacting mass.

**Patentansprüche**

1. Verfahren zur Herstellung von 3,5-Diensäuren mit Doppelbindungen in konjugierter Stellung, dadurch gekennzeichnet, daß ein Vinylhalogenid der Formel:

$$R—CH=CHX,$$

in der R für ein Wasserstoffatom oder eine Hydrocarbylgruppe mit bis zu 20 Kohlenstoffatomen steht, die gegebenenfalls durch unter den Reaktionsbedingungen inerte Substituenten substituiert ist, und X ein Halogen, ausgewählt unter Chlor und Brom ist, mit einem Salz von 3-Butensäure der Formel:

$$CH_2=CH—CH_2—COOM,$$

in der M ein Metall, ausgewählt unter Na, K, 1/2 Ca, 1/2 Mg, oder eine $NR'_4$-Gruppe darstellt, in der R' Alkylgruppen bedeutet, die unabhängig bis zu 12 Kohlenstoffatome haben, in einem organischen Lösungsmittel, das befähigt ist, entweder allein oder im Gemisch mit geringen Wassermengen die Salze von 3-Butensäure zumindest teilweise zu lösen, unter einer Inertatmosphäre bei einer Temperatur zwischen 50°C und 100°C im Falle der Verwendung von komplexierten Phosphinkatalysatoren von Rhodium und zwischen 70°C und 120°C im Falle der Verwendung von komplexierten Phosphinkatalysatoren von Nickel bei Atmosphärendruck in Gegenwart eines Katalysators, ausgewählt unter Phosphinkomplexen von Rhodium und Nickel der Formeln:

$$RhCl(PR''_3)_x \cdot (ol)_y \text{ und } Ni(PR''_3)_z$$

wobei R″ für eine Hydrocarbylgruppe mit bis zu 8 Kohlenstoffatomen steht;

"ol" ein einfaches Olefin mit 2 bis 8 Kohlenstoffatomen oder ein chelatisierendes Olefin mit 6 bis 8 Kohlenstoffatomen bedeutet und wobei

x eine ganze Zahl von 1 bis 3 ist,

y eine ganze Zahl von 0 bis 2 ist, vorausgesetzt daß

$$x+y=3,$$

und z eine ganze Zahl von 3 bis 4 ist, umgesetzt wird.

2. Verfahren nach Anspruch 1, dadurch gekennzeichnet, daß das organische Lösungsmittel eine Verbindung, ausgewählt unter Kohlenwasserstoffen, Ethern, Estern, Nitrilen, Amiden, Alkoholen und Ketonen, vorzugsweise mit einem Siedepunkt unter 180°C ist.

3. Verfahren nach Anspruch 2, dadurch gekennzeichnet, daß das organische Lösungsmittel ausgewählt ist unter Ethanol, Butylether, Anisol, Ethylacetat, Acetonitril, Dimethylformamid, Aceton, Methylethylketon, Toluol.

4. Verfahren nach den Ansprüchen 2 und 3, dadurch gekennzeichnet, daß das organische Lösungsmittel im Gemisch mit geringen Wassermengen verwendet wird.

5. Verfahren nach irgendeinem der vorangehenden Ansprüche, dadurch gekennzeichnet, daß der Rhodiumkatalysator oder Nickelkatalysator gemäß Anspruch 1 ein Komplex mit einem Phosphin $P(R'')_3$ ist, worin R″ ausgewählt ist unter Butyl-, Phenyl- und Anisylgruppen und worin das Olefin "ol" ausgewählt ist unter Ethylen, Cycloocten, 1,5-Hexadien und 1,5-Cyclooctadien.

6. Verfahren nach irgendeinem der vorangehenden Ansprüche, dadurch gekennzeichnet, daß das Vinylhalogenid ausgewählt ist unter Vinylbromid und Styrylbromid.

7. Verfahren nach irgendeinem der vorangehenden Ansprüche, dadurch gekennzeichnet, daß der Katalysator ausgewählt ist unter

$$RhCl(PPh_3)_3 \text{ und } Ni(PPh_3)_3.$$

8. Verfahren nach irgendeinem der vorangehenden Ansprüche, dadurch gekennzeichnet, daß das Vinylhalogenid und das Salz der 3-Butensäure in im wesentlichen äquimolaren Verhältnissen umgesetzt werden.

9. Verfahren nach irgendeinem der vorangehenden Ansprüche, dadurch gekennzeichnet, daß der komplexierte Rhodium- oder Nickelkatalysator in Mengen von mindestens entsprechend 0,1 Millimol pro Liter Reaktionsmasse bis zu 100 Millimol pro Liter Reaktionsmasse verwendet werden.

**Revendications**

1. Procédé de préparation d'acides 3,5-diénoïques comportant des doubles liaisons en position conjuguée, caractérisé en ce qu'un halogénure de vinyle de formule:

$$R—CH=CHX,$$

dans laquelle:

R est un atome d'hydrogène ou un groupe hydrocarboné comprenant jusqu'à 20 atomes de carbone, éventuellement substitué par des substituents inertes dans les conditions réactionnelles, et

X est un halogène consistant en chlore ou brome, est amené à réagir avec un sel d'acide 3-buténoïque ayant la formule:

$$CH_2=CH—CH_2—COOM,$$

dans laquelle:

M est un métal consistant en Na K, $\frac{1}{2}$ Ca, $\frac{1}{2}$ Mg, ou un groupe $NR'_4$ dans lequel R' sont des radicaux alkyles comprenant indépendamment l'un de l'autre jusqu'à 12 atomes de carbone, dans un solvant organique capable de dissoudre au moins partiellement, soit seul soit en mélange avec de faiblesquantités d'eau, les sels d'acide 3-buténoïque, et sous atmosphère inerte, à une température comprise entre 50 et 100°C dans le cas de l'emploi de catalyseurs phosphiniques de complexe de rhodium, et entre 70 et 120°C dans le cas de l'emploi de catalyseurs phosphoniques complexes de nickel

et sous pression atmosphérique, en présence de catalyseur consistant en complexes phosphiniques de rhodium et de nickel, ayant les formules:

$$RhCl(PR''_3)_x \cdot (ol)_y$$

et

$$Ni(PR''_3)_z$$

dans lesquelles:

R″ correspond à un groupe hydrocarboné ayant jusqu'à 8 atomes de carbone,

"ol" représente une simple oléfine ayant 2 à 8 atomes de carbone ou une oléfine chélatante comprenant 6 à 8 atomes de carbone, et

x est un nombre entier compris entre 1 et 3,

y est un nombre entier compris entre 0 et 2, étant entendu que

$$x+y=3,$$

et z étant un nombre entier compris entre 3 et 4.

2. Procédé selon la revendication 1, caractérisé en ce que le solvant organique est un composé choisi dans le groupe des hydrocarbures, éthers, esters, nitriles, amides, alcools et cétones, de préférence présentant un point d'ébullition inférieur à 180°C.

3. Procédé selon la revendication 2, caractérisé en ce que le solvant organique est choisi dans le groupe comprenant l'alcool éthylique, l'éther butylique, l'anisole, l'acétate d'éthyle, l'acétonitrile, le diméthylformamide, l'acétone, la méthyléthylacétone, le toluéne.

4. Procédé selon l'une des revendications 2 et 3, caractérisé en ce que le solvant organique est employé en mélange avec des petites quantités d'eau.

5. Procédé selon l'une quelconque des revendications précédentes, caractérisé en ce que le catalyseur de rhodium ou le catalyseur de nickel, tel que défini dans la revendication 1, consiste en un complexe avec une phosphine de formule:

$$P(R'')_3$$

dans laquelle R″ est choisi dans le groupe des radicaux butyle, phényle et anisyle et où l'oléfine "ol" est choisie dans le groupe comprenant l'éthylène, le cyclooctène, le 1,5-hexadiène et le 1,5-cyclooctadiène,

6. Procédé selon l'une quelconque des revendications précédentes, caractérisé en ce que l'halogènure de vinyle consiste en bromure de vinyle ou bromure de styryle.

7. Procédé selon l'une quelconque des revendications précédentes, caractérisé en ce que le catalyseur consiste en:

$$RhCl(PPh_3)_3$$

et

$$Ni(PPh_3)_3.$$

8. Procédé selon l'une quelconque des revendications précédentes, caractérisé en ce que l'halogènure de vinyl et le sel de l'acide 3-buténoïque sont amenés à réagir en proportions sensiblement équimolaires.

9. Procédé selon l'une quelconque des revendications précédentes, caractérisé en ce que le catalyseur complexe de rhodium ou de nickel est utilisé en quantités au moins égales à 0,1 millimole par litre de masse réagissante, et pouvant atteindre jusqu'à 100 millimoles par litre de masse réagissante.